# EUROPÄISCHE PATENTSCHRIFT

(11) **EP 3 757 232 B1**
(45) Veröffentlichungstag und Bekanntmachung des Hinweises auf die Patenterteilung: **07.06.2023**
(21) Anmeldenummer: 20176259.8
(22) Anmeldetag: 25.05.2020
(51) Int. Cl.: C14B 1/26, C14B 17/08

(54) **VORRICHTUNG ZUM STRECKEN VON TIERHAUT**
DEVICE FOR STRETCHING ANIMAL SKIN
DISPOSITIF D'ÉTIREMENT DE LA PEAU ANIMALE

(30) Priorität: 24.06.2019 AT 505572019
(43) Veröffentlichungstag der Anmeldung: 30.12.2020
(73) Patentinhaber: Wollsdorf International GmbH, 8181 Wollsdorf (AT)
(72) Erfinder: Hitrec, Dean, 49 250 Zlatar (HR); Laskowska, Katarzyna, 8200 Gleisdorf (AT)
(74) Vertreter: Röggla, Harald

(56) Entgegenhaltungen:
- WO-A1-96/06192
- WO-A2-2016/014533
- US-A- 4 335 594

## Beschreibung

Die Erfindung betrifft eine Vorrichtung zum Strecken von Tierhaut.

Bei der Lederherstellung wird in einer Abfolge von mechanischen und chemischen Prozessen aus einer Tierhaut eine fertige Lederhaut erzeugt. Da Leder in einer Vielzahl unterschiedlicher Anwendungen eingesetzt wird, muss vor bzw. nach verschiedenen Prozessschritten während der Lederherstellung die Qualität der Tierhaut bestimmt werden, um festzulegen, für welche Anwendung die fertige Lederhaut oder Teilbereiche der Lederhaut geeignet sind. Der Wert einer Lederhaut ist ebenfalls wesentlich durch die Qualität der fertigen Lederhaut geprägt. Die Inspektion der Lederhäute erfolgt nach dem Stand der Technik mittels visuellen Inspektionsmethoden, wobei die Inspektion entweder manuell von einem Menschen oder automatisiert, mittels eines Kamerasystems durchgeführt wird. Bei der Qualitätskontrolle von Tierhaut kommen mehrere unterschiedliche Prüfeinrichtungen zur Prüfung unterschiedlicher Qualitätskriterien zum Einsatz. Mit einem so genannten Taber bzw. Veslic oder Martindale Prüfgerät wird die Abriebfestigkeit geprüft. Mit einem so genannten Flexometer wird die Knickbeständigkeit geprüft. Bei einem Flammtest wird die Enflammbarkeit geprüft. Zu Leder veredelte Tierhaut wird Licht und UV-Strahlung ausgesetzt, um die Lichtechtheit zu prüfen. Mit einem weiteren Gerät wird eine Farbmessung der Farbe der Oberfläche der Tierhaut durchgeführt. Des Weiteren wird die zu Leder fertig verarbeitete Tierhaut auf Festigkeit (z.B. Zugfestigkeit, Weiterreisskraft, Dehnbarkeit) geprüft. Abschießend findet eine manuelle und visuelle Endkontrolle statt, bei der im Wesentlichen die Qualität bezüglich Hautschädigungen des Leders als Qualitätskriterium beurteilt wird.

Bei beispielsweise Rindsleder sind eine Vielzahl unterschiedlicher Arten von Hautschädigungen bekannt, die auf Verletzungen, Flechten, Milben, Risse, Gabelstöße und andere Ursachen zurückzuführen sind. Jede Art dieser Hautschädigungen hat eine typische Ausbildung, wobei Gabelstöße beispielsweise einen kleinen runden Umfang und Hautschädigungen durch Mastfalten längliche und parallel verlaufende Umrisse aufweisen. Vorrichtungen zum Strecken von Tierhaut werden in der Lederproduktion im Rahmen der Qualitätskontrolle eingesetzt, um Schädigungen an Tierhäuten einfacher zu erkennen. Hierbei wird die Tierhaut gestreckt, wodurch die oben genannten Schädigungen beziehungsweise Qualitätsmerkmale einfacher mit im Stand der Technik bekannten Prüfvorrichtungen bestimmt werden können. Hierdurch erhöht sich die Genauigkeit der Qualitätskontrolle in der Lederherstellung.

Aus dem Stand der Technik sind sogenannte Spanntische mit einer manuellen Bedienung bekannt. Bei diesen wird die Haut mit einem Spannrahmen, der eine Klemmung eines Hautbereiches bewirkt befestigt, und dann durch einen beweglichen Hubkern, der durch seine Bewegung den fixierten Bereich von der Rückseite der Tierhaut dehnt, gespannt. Je nach Hubhöhe, kann so eine Prozentrate einer gewünschten Dehnung dargestellt werden. Die Inspektion und Markierung mit einem Farbstift der Ledermerkmale erfolgt rein manuell.

Aus der AT 509 382 A1 ist eine Vorrichtung zum Strecken von Tierhaut bekannt, welche mittels Zugmitteln, welche mit einer linear wirkenden Zugspannung beaufschlagt werden, die Tierhaut für eine nachfolgende Qualitätskontrolle streckt. In dieser, aus dem Stand der Technik vorbekannten Vorrichtung wird eine möglichst homogene Streckung der Tierhaut erreicht, indem die Zugmittel die Tierhaut entlang von zwei aufeinander normal stehenden Zugspannungslinien strecken, mit dem Ziel eine möglichst große Zahl an Qualitätsmängeln in der Tierhaut zu erkennen. Aufgrund der vielfältigen Natur der in Tierhäuten auftretenden Schädigungen werden jedoch manche Schädigungen im Zuge der nachfolgenden Qualitätskontrolle dennoch nicht detektiert. Des Weiteren kann mit der in der AT 509 382 A1 offenbarten Vorrichtung die Tierhaut in vielen Randbereichen nicht in alle Richtungen gestreckt werden wodurch eine Untersuchung der gesamten Tierhaut in einem Arbeitsschritt verunmöglicht wird. Außerdem ermöglicht dieses vorbekannte Spannsystem keinerlei Einschätzung der zu erwartenden Dehnungswerte, die diese Haut beim späteren Verarbeiten in Einzelzuschnitte haben wird

Das Dokument WO 96/06192 A1 offenbart ein Verfahren und eine Vorrichtung zur Verarbeitung von Tierhäuten, mit einem Spannrahmen zur Aufnahme der Tierhäute.

Das Dokument US 4,335,594 offenbart eine Vorrichtung zum Spannen von Tierhäuten welche einen Spannrahmen aufweist, welcher eine Anzahl an zueinander beabstandeten, radialen Gleitführungen umfasst.

Das Dokument WO 2016/014533 A2 offenbart eine Vorrichtung und ein Verfahren zur Fixierung von Geweben, die in bioprothetischen Herzklappen verwendet werden, wobei die Vorrichtung einen Spannrahmen umfasst.

Es ist die Aufgabe der vorliegenden Erfindung eine Vorrichtung zum Strecken von Tierhaut zu bilden, welche die Nachteile des Standes der Technik vermeidet.

Erfindungsgemäß wird die vorliegende Aufgabe mit einer Vorrichtung zum Strecken von Tierhaut mit den Merkmalen von Anspruch 1 gelöst.

Die erfindungsgemäße Vorrichtung zum Strecken von Tierhaut umfasst eine Auflagefläche zur Auflage der Tierhaut, sowie ein erstes Paar, und ein zweites Paar an im Wesentlichen an gegenüberliegenden Randbereichen der Tierhaut befestigbaren Zugmitteln. Die Zugmittel sind mit einer Spannvorrichtung zum Beaufschlagen der Zugmittel mit einer Zugspannung verbunden, wobei jedes Paar an Zugmitteln bei einer Beaufschlagung mit der Zugspannung eine Zugspannungslinie in der Tierhaut bildet. Erfindungsgemäß schließt die Zugspannungslinie des ersten Paares mit der Zugspannungslinie des zweiten Paares einen stumpfen und/oder spitzen Winkel ein. Durch die Bildung eines stumpfen und/oder spitzen Winkels zwischen den Zugspannungslinien wird eine inhomogene Streckung der Tierhaut erreicht. Überraschenderweise ergibt sich durch eine inhomogene Streckung der Tierhaut der technische Effekt, dass abhängig von den Streckrichtungen beziehungsweise der Geometrie der Zugspannungslinien eine größere Anzahl an Qualitätsmängeln und Schädigungen in der Tierhaut im Zuge der Qualitätskontrolle erkennbar ist, als bei einer im Stand der Technik bekannten homogenen Streckung, oder einer Streckung entlang nur einer einzelnen Zugspannungslinie. Hierdurch wird eine Verbesserung der Erkennbarkeit von Schädigungen in der Tierhaut erreicht, wodurch die Qualität der Lederproduktion insgesamt gesteigert wird.

Gemäß der bevorzugten Ausführungsform der erfindungsgemäßen Vorrichtung umfasst die Vorrichtung zumindest ein weiteres Paar an im Wesentlichen an gegenüberliegenden Randbereichen der Tierhaut befestigbaren Zugmitteln, wobei die Zugspannungslinie jedes Paares an Zugmitteln mit der Zugspannungslinie eines benachbarten Paares an Zugmitteln einen stumpfen und/oder spitzen Winkel einschließt. Hierdurch wird der Vorteil erreicht, dass weitere Möglichkeiten zur Variation der Streckung der Tierhaut bereitgestellt werden, wodurch die Anzahl an Defekten in der Tierhaut, welche mit der erfindungsgemäßen Vorrichtung sichtbar gemacht werden können, erhöht wird. Selbst bei einer nachfolgenden, rein visuellen Inspektion der Tierhaut wird hierdurch ein deutlich höherer Erkennungsgrad erreicht, als mit im Stand der Technik üblichen Inspektionstischen.

Vorzugsweise schneiden sich die Zugspannungslinien der Paare an Zugmitteln im Wesentlichen in einem Zentralbereich der Tierhaut. Hierdurch wird der Vorteil erreicht, dass ein möglichst großer Bereich der Tierhaut mit der erfindungsgemäßen Vorrichtung gestreckt werden kann. Die ganze Tierhaut kann so in einer gesamthaften Aufspannung ohne weiteres Umsetzen von Teilbereichen auf Ledermerkmale inspeziert werden. In Kombination mit einer, durch einer Person durchgeführten visuellen Kontrolle oder durch Einsatz eines geeigneten Kamerasystems ergibt sich zu am Markt erhältlichen Systemen der klare Vorteil, dass die komplette Tierhaut in einer Auspannungslage ohne weiteren Materialtransport verarbeitbar wird.

Die erfindungsgemäße Vorrichtung umfasst einen ersten Anschlag und einen zweiten Anschlag. Die Spannvorrichtung ist zwischen dem ersten Anschlag und dem zweiten Anschlag beweglich gelagert, wobei die Zugmittel, wenn die Spannvorrichtung am ersten Anschlag anliegt, im Wesentlichen zugspannungsfrei, und wenn die Spannvorrichtung am zweiten Anschlag anliegt, mit der Zugspannung beaufschlagt sind. Durch die Anschläge wird ein Bereich vorgegeben, innerhalb dessen die Tierhaut gedehnt werden kann. Hierdurch wird verhindert, dass die Tierhaut überdehnt oder gar mechanisch in ihrer Struktur beschädigt wird . Vorzugsweise ist der Abstand zwischen dem ersten Anschlag und dem zweiten Anschlag variabel. Da die Häute unterschiedlicher Tierarten über unterschiedliche Elastizitätsgrenzen verfügen bietet dies bietet die Möglichkeit die Größe der Dehnung, welche auf die Tierhaut mit der erfindungsgemäßen Vorrichtung aufgebracht wird, an den Typ der Tierhaut anzupassen. Außerdem kann hierdurch auch die Dehnbarkeit der Tierhaut als Wert klassifiziert werden, was mit anderen Methoden des Standes der Technik unmöglich ist.

Erfindungsgemäß ist die Spannvorrichtung mit einem Seilzug verbunden, wobei der Seilzug dazu ausgebildet ist die Spannvorrichtung zwischen dem ersten Anschlag und dem zweiten Anschlag zu verlagern. Hierdurch wird der Vorteil erreicht, dass eine am Markt einfach und für verschiedene Lastbereiche verfügbares Mittel für die erfindungsgemäße Vorrichtung verwendet werden kann. Vorzugsweise umfasst die erfindungsgemäße Vorrichtung zwei mit der Spannvorrichtung verbundene Seilzüge.

Gemäß der bevorzugten Ausführungsform der erfindungsgemäßen Vorrichtung umfasst die Spannvorrichtung zumindest ein Aufnahmemittel zur Aufnahme von Spanngewichten. Hierdurch wird der Vorteil erreicht, dass die erfindungsgemäße Vorrichtung auch ohne den Einsatz einer externen Energiequelle beziehungsweise Spannungsversorgung betrieben werden kann. Vorzugsweise ist das Aufnahmemittel als im Wesentlichen kreisförmige Platte ausgebildet. Diese ist vorzugsweise parallel zu der Auflagefläche angeordnet.

Gemäß einer vorteilhaften Ausführungsvariante umfassen die Zugmittel selbstsichernde Zugklemmen zur Befestigung der Zugmittel an der Tierhaut. Hierdurch wird der Vorteil erreicht, dass eine einfache und schnelle Befestigungsmethode bereitgestellt wird, welche eine zu der Zugspannung proportionale Klemmkraft bereitstellt und somit eine sichere Befestigung der Tierhaut an den Zugmitteln ermöglicht. Die Zugklemmen sind konstruktiv so ausgeführt, dass sie Beschädigungen an der aufzuspannenden Haut ausschliessen

Die erfindungsgemäße Vorrichtung umfasst in einer vorteilhaften Ausführungsvariante Umlenkmittel zum Umlenken der Zugmittel. Diese sind vorzugsweise in einem Randbereich der Auflagefläche angeordnet. Hierdurch wird eine kompakte Bauform der erfindungsgemäßen Vorrichtung erreicht.

Vorzugsweise ist die Auflagefläche im Wesentlichen kreisförmig oder als Vieleck ausgeführt. Zudem umfasst die erfindungsgemäße Vorrichtung in der bevorzugten Ausführungsform einen Auflagebock, wobei die die Auflagefläche, die Zugmittel und die Spannvorrichtung mit dem Auflagebock verbunden sind.

Vorteilhafte Ausgestaltungen der erfindungsgemäßen Vorrichtung sowie alternative Ausführungsvarianten werden in weiterer Folge anhand der Figuren näher erläutert.
Figur 1 zeigt die erfindungsgemäße Vorrichtung zum Strecken von Tierhaut mit einer auf einer Auflagefläche der Vorrichtung befestigten Tierhaut in einer Schrägansicht von oben.
Figur 1a zeigt eine selbstsichernde Zugklemme der erfindungsgemäßen Vorrichtung.
Figur 2 zeigt die erfindungsgemäße Vorrichtung in einer Schrägansicht von unten.
Figur 3 zeigt einen ersten Anschlag und einen zweiten Anschlag der erfindungsgemäßen Vorrichtung.
Figur 4 zeigt einen Abschnitt einer Spannvorrichtung der erfindungsgemäßen Vorrichtung.
Figur 5 zeigt die Spannvorrichtung erfindungsgemäßen Vorrichtung mit Spanngewichten.
Figur 6 zeigt einen Verlauf von Zugmitteln der erfindungsgemäßen Vorrichtung.
Figur 7 zeigt einen Auflagebock der erfindungsgemäßen Vorrichtung.
Figur 8 zeigt die erfindungsgemäße Vorrichtung gemäß einer alternativen Ausführungsvariante mit Hydraulikzylindern.
Figur 9 zeigt die erfindungsgemäße Vorrichtung mit einer Auflagefläche mit Vertiefungen.

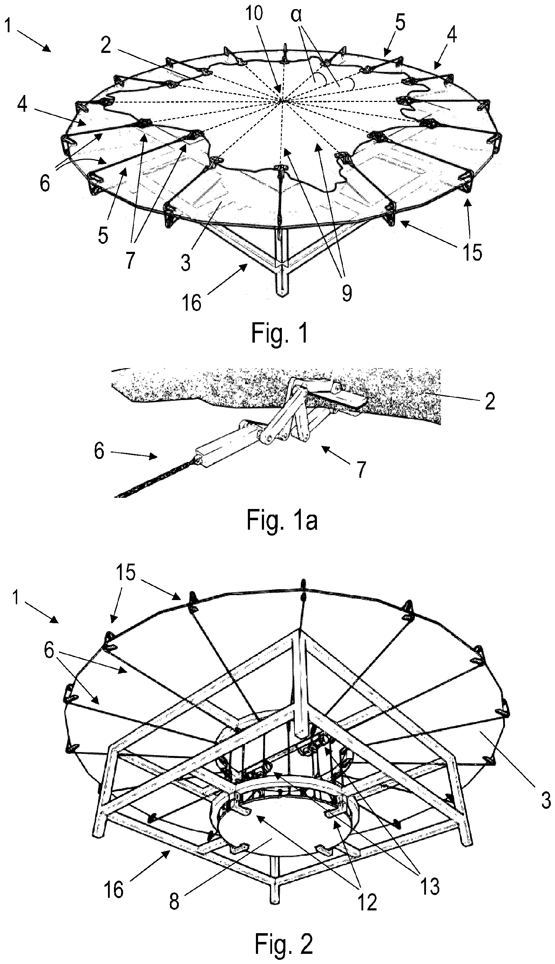

Figur 1 zeigt eine erfindungsgemäße Vorrichtung 1 zum Strecken von Tierhaut 2. Die Vorrichtung 1 umfasst eine Auflagefläche 3, welche zur Auflage der Tierhaut 2 dient. Die Vorrichtung 1 umfasst ein erstes Paar 4 und ein zweites Paar 5 an im Wesentlichen an gegenüberliegenden Randbereichen der Tierhaut 2 befestigbaren Zugmitteln 6. Die in Figur 1 in einer bevorzugten Ausführungsform dargestellte Vorrichtung 1 umfasst acht Paare an Zugmitteln 6, wobei die Zugmittel 6 befestigt an der Tierhaut 2 dargestellt sind. Gemäß einer alternativen Ausführungsvariante der erfindungsgemäßen Vorrichtung 1 können beispielsweise auch zehn bis dreißig Paare an Zugmitteln 6 an der erfindungsgemäßen Vorrichtung 1 eingesetzt werden. Die Paare an Zugmitteln 6 sind vorzugsweise konturumlaufend angeordnet, und setzten im Wesentlichen die gesamte Tierhaut 2 sternförmig auf Zugspannung. Die Zugmittel 6 umfassen vorzugsweise selbstsichernde Zugklemmen 7 zum Befestigen der Zugmittel 6 an der Tierhaut 2. Eine selbstsichernde Zugklemme 7 ist in Figur 1a im Detail dargestellt. Die Zugmittel 6 sind mit einer Spannvorrichtung 8 verbunden, welche in Figur 2, Figur 4 und Figur 5 dargestellt ist. Die Spannvorrichtung 8 stellt eine Zugspannung bereit und beaufschlagt die Zugmittel 6 mit der Zugspannung. Jedes Paar 4 und 5 an mit der Zugspannung beaufschlagten Zugmitteln 6 bildet eine Zugspannungslinie 9 in der Tierhaut 2, welche in Figur 1 als strichlierte Linien dargestellt sind. Erfindungsgemäß schließt die Zugspannungslinie 9 des ersten Paares 4 an Zugmitteln 6 mit der Zugspannungslinie 9 des zweiten Paares 5 an Zugmitteln 6 einen stumpfen und/oder spitzen Winkel α ein. Durch den stumpfen und/oder spitzen Winkel α welchen die Zugspannungslinien 9 des ersten Paares 4 und des zweiten Paares 5 einschließen wird eine inhomogene Streckung in einem Teil- oder im Wesentlichen dem gesamten Bereich der Tierhaut 2 erreicht. Hierdurch ergibt sich der Vorteil, dass die Streckung der Tierhaut 2 an die Art der Qualitätsmängel von Interesse in der Tierhaut 2 selektiv angepasst werden kann. Hierdurch wird die Detektion bestimmter Qualitätsmängel erleichtert, und die Qualitätskontrolle in der Lederproduktion verbessert.

Gemäß der in Figur 1 dargestellten, bevorzugten Ausführungsform der erfindungsgemäßen Vorrichtung 1 umfasst die Vorrichtung 1 zumindest eine weiteres Paar an im Wesentlichen an gegenüberliegenden Randbereichen der Tierhaut 2 befestigbaren Zugmitteln 6. In Figur 1 ist die Vorrichtung mit acht Paaren an Zugmitteln 6 dargestellt. Die Zugspannungslinie 9 jedes Paares an Zugmitteln 6 schließt mit der Zugspannungslinie 9 eines benachbarten Paares an Zugmitteln 6 einen stumpfen und/oder spitzen Winkel α ein. Der stumpfe und/oder spitze Winkel α kann zwischen den Zugspannungslinien 9 aufeinanderfolgender Paare an Zugmitteln 6 variieren. Hierdurch wird der Vorteil erreicht, dass im Wesentlichen die gesamte Fläche der Tierhaut 2 mit der erfindungsgemäßen Vorrichtung 1 gespannt werden kann. Des Weiteren schneiden sich die Zugspannungslinien 9, wie in Figur 1 dargestellt, vorzugsweise im Wesentlichen in einem Zentralbereich 10 der Tierhaut 2. Hierdurch wird der Vorteil erreicht, dass die Tierhaut 2 beim Strecken keine Falten oder Risse bildet und somit beschädigt wird. Gemäß einer bevorzugten Ausführungsvariante der erfindungsgemäßen Vorrichtung 1 umfasst die Vorrichtung 1 sechzehn Paare an Zugmitteln 6. Hierdurch wird die Zugspannung auf die Tierhaut 2 noch gleichmäßiger verteilt, und die Kraft pro Zugmittel 6 reduziert. Hierdurch wird der Vorteil erreicht, dass das Risiko eines Ausreißens einzelner Zugmittel 6 reduziert, und die Sicherheit für das Bedienpersonal erhöht wird.

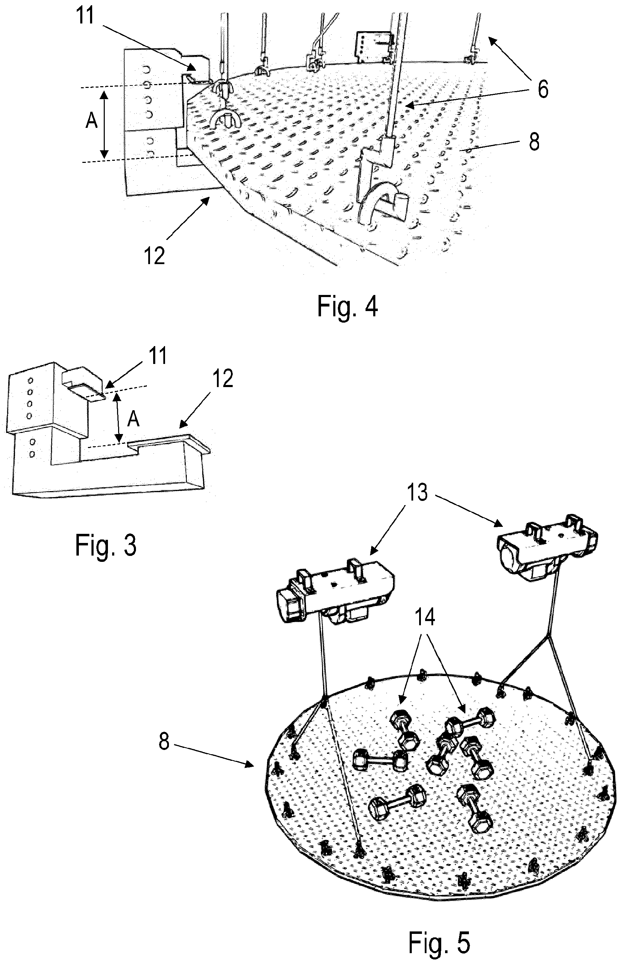

Figur 2 zeigt die erfindungsgemäße Vorrichtung 1 in einer Ansicht von unten, wobei die Spannvorrichtung 8 sichtbar ist. Die Vorrichtung 1 umfasst einen ersten Anschlag 11, welcher in Figur 3 sichtbar ist, und einen zweiten Anschlag 12, zwischen welchen die Spannvorrichtung 8 beweglich gelagert ist. Der erste Anschlag 11 und der zweite Anschlag 12 sind in Figur 3 im Detail dargestellt. Wie aus Figur 3 ersichtlich ist in der bevorzugten Ausführungsform der erfindungsgemäßen Vorrichtung 1 ein Abstand A zwischen dem ersten Anschlag 11 und dem zweiten Anschlag 12 variabel. Die Zugmittel 6 sind, wenn die Spannvorrichtung 1 am ersten Anschlag 11 anliegt, im Wesentlichen zugspannungsfrei, und wenn die Spannvorrichtung 8 am zweiten Anschlag 12 anliegt, mit der Zugspannung beaufschlagt. Durch die Variabilität des Abstands A wird der Vorteil erreicht, dass die Zugspannung an Tierhäute 2 unterschiedlicher Tierarten angepasst werden kann. In Figur 4 ist die Spannvorrichtung 8 abschnittsweise dargestellt, wobei die Spannvorrichtung 8 in Figur 4 am zweiten Anschlag 12 anliegt, und die Zugmittel 6 mit der Zugspannung beaufschlagt sind. Durch den ersten Anschlag 11 und den zweiten Anschlag 12 wird die maximale Zugspannung begrenzt, welche auf die Tierhaut 2 mit der erfindungsgemäßen Vorrichtung 1 aufgebracht wird.

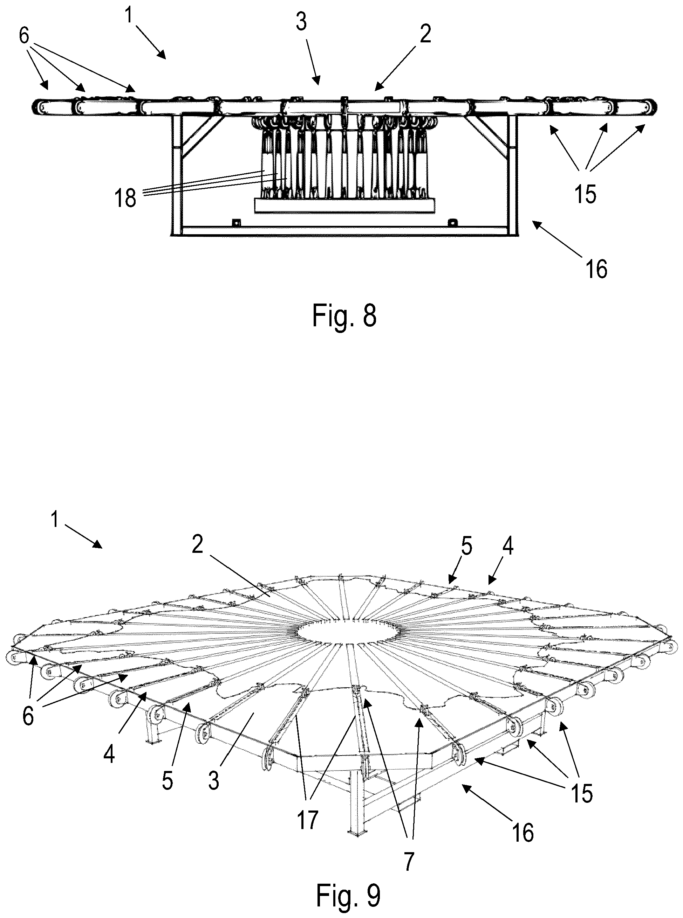

Figur 5 zeigt die Spannvorrichtung 8 der erfindungsgemäßen Vorrichtung 1, welche mit einem Seilzug 13 verbunden ist. Gemäß der bevorzugten Ausführungsform der erfindungsgemäßen Vorrichtung 1 umfasst die Vorrichtung 1, wie in Figur 5 dargestellt, zwei mit der Spannvorrichtung 8 verbundene Seilzüge 13. Der beziehungsweise die Seilzüge 13 verlagern die Spannvorrichtung 8 zwischen dem ersten Anschlag 11 und dem zweiten Anschlag 12. Vorzugweise umfasst die Spannvorrichtung 8 ein Aufnahmemittel zur Aufnahme von Spanngewichten 14. Hierdurch wird der Vorteil erreicht, dass die Zugspannung durch die Gewichtskraft der Spanngewichte 14 bereitgestellt wird, wobei die Seilzüge 13 die Spanngewichte 14 in Richtung des ersten Anschlags 11 anheben beziehungsweise die Spannvorrichtung 8 durch die Spanngewichte 14 in Richtung des zweiten Anschlags 12 abgesenkt wird. Gemäß einer alternativen Ausführungsvariante kann der, beziehungsweise können die Seilzüge 13 auch anstatt der Spanngewichte 14 eingesetzt werden, um eine Kraft zur Verlagerung der Spannvorrichtung 8 zwischen dem ersten Anschlag 11 und den zweiten Anschlag 12 bereitzustellen. Durch eine Umrechnung der aufgebrachten Kräfte kann auch eine klare Vorhersage über die zu erwartenden Dehnungseigenschaften der Tierhaut 2 getroffen werden. Dies erleichtert die Auswahl von Tierhäuten 2 für den späteren Zuschnitt, wenn aus verschiedenen Tierhäuten 2 Zuschnittsteile entstehen müssen, die als kommissioniertes Set bestehend aus mehreren Einzelteilen gleiche Dehnungswerte aufweisen müssen. Des Weiteren können, wie in Figur 8 dargestellt, anstatt der oder zusätzlich zu den Spanngewichten 14 auch mit den Zugmitteln 6 verbundene Hydraulikzylinder 18 eingesetzt werden, welche sowohl eine Bestimmung der aufgebrachten Kraft als auch der Wegstrecke ermöglichen, und somit eine Berechnung der, auf die Tierhaut 2 aufgebrachten Zugspannung ermöglichen. Eine Verwendung von Hydraulikzylindern 18 anstatt oder zusätzlich zu den Spanngewichten 2 ermöglicht zudem auf den ersten Anschlag 11 und den zweiten Anschlag 12 zu verzichten, und die Längsdehnung beziehungsweise Kontraktion der Hydraulikzylinder 18 direkt an dem jeweiligen Hydraulikzylinder 18 oder mittels einer gesonderten Steuer- beziehungsweise Messeinrichtung zu bestimmen. Alternativ können auch an der Vorrichtung 1 mit Hydraulikzylindern 18 der erste Anschlag 11 und der zweite Anschlag 12 vorgesehen sein. Der Druck innerhalb der Hydraulikzylinder 18 kann zudem als Indikator für die Elastizität der Tierhaut 2 herangezogen werden. Zudem können die Hydraulikzylinder 18 Blockierventile umfassen, welche eine Länge der Hydraulikzylinder fixieren. Alternativ können auch alle Zugmittel 6 mit zumindest einem Hydraulikzylinder 18 verbunden sein. Des Weiteren kann wie in Figur 8 gezeigt jedes Zugmittel 6 mit einem eigenen Hydraulikzylinder 18 zum Aufbringen von Zugspannung auf das Zugmittel 6 verbunden sein. Zusätzlich zu diesen kann ein, in Figur 8 nicht dargestellter, zentraler, zusätzlicher Haupthydraulikzylinder vorgesehen sein, welcher mit den Hydraulikzylindern 18 verbunden ist, und nach einer, vorzugsweise gleichmäßigen Spannung der Zugmittel 6 mittels der den einzelnen Zugmitteln 6 zugeordneten Hydraulikzylindern 18, eine zentrale Kraft auf alle Zugmittel 6 gleichzeitig aufbringt.

Das Aufnahmemittel ist vorzugsweise, wie in Figur 5 dargestellt, als in Wesentlichen kreisrunde Platte ausgeführt. Die kreisrunde Platte ist gemäß eine bevorzugten Ausführungsvariante Wesentlichen parallel zu der Auflagefläche 3 angeordnet. Zudem ist die Auflagefläche 3 vorzugsweise im Wesentlichen kreisförmig. Hierdurch wird eine gleichmäßige Spannung der Zugmittel 6 gewährleistet. Gemäß alternativen Ausführungsvarianten kann die Auflagefläche 3, wie in Figur 9 dargestellt, auch quadratisch oder im Wesentlichen quadratisch mit runden oder abgefasten Ecken ausgeführt sein.

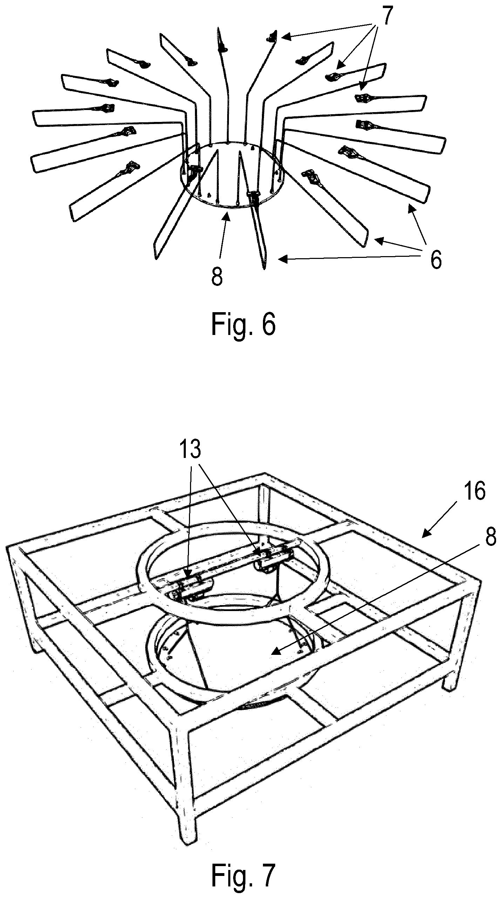

Wie in Figur 1 und Figur 2 dargestellt umfasst die Vorrichtung 1 Umlenkmittel 15 zum Umlenken der Zugmittel 6. Figur 6 zeigt den Verlauf der Zugmittel 6 und die Spannvorrichtung 8 der erfindungsgemäßen Vorrichtung in der bevorzugten Ausführungsform. Die Umlenkmittel 15 führen die Zugmittel 6 entlang des in Figur 6 dargestellten Verlaufs von der Auflagefläche 3 zu der Spannvorrichtung 8. Vorzugsweise sind die Umlenkmittel 15 an einem Randbereich der Auflagefläche 3 angeordnet. Die Zugmittel 6 werden mittels der Umlenkmittel 15 mehrmals umgelenkt, um die Zugspannung von der Spannvorrichtung 8 auf die Tierhaut 2 zu übertragen.

Vorzugweise umfasst die Vorrichtung 1 einen in Figur 2 und in Figur 7 im Detail dargestellten Auflagebock 16. Gemäß der bevorzugten Ausführungsvariante der erfindungsgemäßen Vorrichtung 1 sind die Auflagefläche 2, die Zugmittel 6 und die Spannvorrichtung 8 mit dem Auflagebock 16 verbunden. Des Weiteren können der erste Anschlag 11, der zweite Anschlag 12, die Umlenkmittel 15 und die Seilzüge 13 mit dem Auflagebock 16 verbunden sein.

Gemäß einer, in Figur 9 dargestellten, alternativen Ausführungsvariante der erfindungsgemäßen Vorrichtung 1 umfasst die Auflagefläche 3 Vertiefungen 17, wobei die Zugmittel 6 zumindest teilweise in den Vertiefungen 17 aufgenommen sind. Hierdurch bilden die Zugmittel 6 mit der Auflagefläche 3 im Wesentlichen eine Ebene, wodurch der Vorteil erreicht wird, dass die Tierhaut 2 beim Aufbringen einer Zugspannung nicht von der Auflagefläche 3 abgehoben wird. Hierdurch wird eine verbesserte Detektion von Bereichen mit inhomogener Spannung beziehungsweise Zugfestigkeit in der Tierhaut 2 erreicht. Vorzugsweise sind zudem die Umlenkmittel 15 und/oder die Zugklemmen 7 zumindest teilweise in den Vertiefungen aufgenommen. Die Vertiefungen 17 sind vorteilhaft als Führungsrillen ausgebildet, wodurch eine reproduzierbare, und für jede Tierhaut 2 im Wesentlichen gleiche Klemmung beziehungsweise ein im Wesentlichen gleicher Klemmwinkel und eine im Wesentlichen gleiche Konfiguration der Zugspannungslinien 9 zueinander erreicht wird.

## Patentansprüche

1. Vorrichtung (1) zum Strecken von Tierhaut (2), mit einer Auflagefläche (3) zur Auflage der Tierhaut (2), einem ersten Paar (4), und einem zweiten Paar (5) an im Wesentlichen an gegenüberliegenden Randbereichen der Tierhaut (2) befestigbaren Zugmitteln (6), wobei die Zugmittel (6) mit einer Spannvorrichtung (8) zum Beaufschlagen der Zugmittel (6) mit einer Zugspannung verbunden sind, und jedes Paar (4, 5) an Zugmitteln (6) bei einer Beaufschlagung mit der Zugspannung eine Zugspannungslinie (9) in der Tierhaut (2) bildet, wobei die Zugspannungslinie (9) des ersten Paares (4) mit der Zugspannungslinie (9) des zweiten Paares (5) einen spitzen Winkel (α) einschließt, **dadurch gekennzeichnet, dass** die Vorrichtung (1) einen ersten Anschlag (11) und einen zweiten Anschlag (12) umfasst, und die Spannvorrichtung (8) zwischen dem ersten Anschlag (11) und dem zweiten Anschlag (12) beweglich gelagert ist, wobei die Zugmittel (6), wenn die Spannvorrichtung (8) am ersten Anschlag (11) anliegt, im Wesentlichen zugspannungsfrei, und wenn die Spannvorrichtung (8) am zweiten Anschlag (12) anliegt, mit der Zugspannung beaufschlagt sind.

2. Vorrichtung (1) gemäß Anspruch 1, **dadurch gekennzeichnet, dass** die Vorrichtung (1) zumindest ein weiteres Paar an im Wesentlichen an gegenüberliegenden Randbereichen der Tierhaut (2) befestigbaren Zugmitteln (6) umfasst, wobei die Zugspannungslinie (9) jedes Paares an Zugmitteln (6) mit der Zugspannungslinie (9) eines benachbarten Paares an Zugmitteln (6) einen spitzen Winkel (α) einschließt.

3. Vorrichtung (1) gemäß einem der Ansprüche 1 oder 2, **dadurch gekennzeichnet, dass** sich die Zugspannungslinien (9) der Paare (4, 5) an Zugmitteln (6) im Wesentlichen in einem Zentralbereich (10) der Tierhaut (2) schneiden.

4. Vorrichtung (1) gemäß einem der Ansprüche 1 bis 3, **dadurch gekennzeichnet, dass** ein Abstand zwischen dem ersten Anschlag (11) und dem zweiten Anschlag (12) variabel ist.

5. Vorrichtung (1) gemäß einem der Ansprüche 1 bis 4, **dadurch gekennzeichnet, dass** die Spannvorrichtung (8) mit einem Seilzug (13) verbunden ist, wobei der Seilzug (13) dazu ausgebildet ist die Spannvorrichtung (8) zwischen dem ersten Anschlag (11) und dem zweiten Anschlag (12) zu verlagern.

6. Vorrichtung (1) gemäß einem der Ansprüche 1 bis 5 **dadurch gekennzeichnet, dass** die Spannvorrichtung (8) zumindest ein Aufnahmemittel zur Aufnahme von Spanngewichten (14) umfasst.

7. Vorrichtung (1) gemäß Anspruch 6, **dadurch gekennzeichnet, dass** das Aufnahmemittel als im Wesentlichen kreisförmige Platte ausgebildet ist.

8. Vorrichtung (1) gemäß Anspruch 7, **dadurch gekennzeichnet, dass** die Platte im Wesentlichen parallel zu der Auflagefläche (3) angeordnet ist.

9. Vorrichtung (1) gemäß einem der Ansprüche 1 bis 8, **dadurch gekennzeichnet, dass** die Zugmittel (6) selbstsichernde Zugklemmen (7) zur Befestigung der Zugmittel (6) an der Tierhaut (2) umfassen.

10. Vorrichtung (1) gemäß einem der Ansprüche 1 bis 9, **dadurch gekennzeichnet, dass** die Vorrichtung (1) Umlenkmittel (15) zum Umlenken der Zugmittel (6) umfasst.

11. Vorrichtung (1) gemäß Anspruch 10, **dadurch gekennzeichnet, dass** die Umlenkmittel (15) an einem Randbereich der Auflagefläche (3) angeordnet sind.

12. Vorrichtung (1) gemäß Anspruch 5, **dadurch gekennzeichnet, dass** die die Spannvorrichtung (8) mit zwei Seilzügen (13) verbunden ist.

13. Vorrichtung (1) gemäß einem der Ansprüche 1 bis 12, **dadurch gekennzeichnet, dass** die Vorrichtung (1) einen Auflagebock (16) umfasst, wobei die Auflagefläche (3), die Zugmittel (6) und die Spannvorrichtung (8) mit dem Auflagebock (16) verbunden sind.

14. Vorrichtung (1) gemäß einem der Ansprüche 1 bis 13, **dadurch gekennzeichnet, dass** die Auflagefläche (3) im Wesentlichen kreisförmig ist.

15. Vorrichtung (1) gemäß einem der Ansprüche 1 bis 14, **dadurch gekennzeichnet, dass** die Auflagefläche (3) Vertiefungen (17) umfasst, wobei die Zugmittel (6), die Zugklemmen (7) und/oder die Umlenkmittel (15) zumindest teilweise in den Vertiefungen (17) aufgenommen sind.

16. Vorrichtung (1) gemäß einem der Ansprüche 1 bis 15, **dadurch gekennzeichnet, dass** die Zugmittel (6) mit zumindest einem Hydraulikzylinder (18) verbunden sind.

## Claims

1. A device (1) for stretching animal hide (2), comprising a bearing surface (3) for supporting the animal hide (2), a first pair (4) and a second pair (5) of traction means (6) essentially attachable to opposite edge areas of the animal hide (2), the traction means (6) being connected to a tensioning device (8) for charging the traction means (6) with a tensile stress, and each pair (4, 5) of traction means (6) forming a tensile stress line (9) in the animal hide (2) when being charged with the tensile stress, the tensile stress line (9) of the first pair (4) enclosing an acute angle (α) with the tensile stress line (9) of the second pair (5), characterized that the device (1) comprises a first stop (11) and a second stop (12), and the tensioning device (8) is movably mounted between the first stop (11) and the second stop (12), with the traction means (6) being essentially free of tensile stress when the tensioning device (8) rests against the first stop (11), and being charged with the tensile stress when the tensioning device (8) rests against the second stop (12).

2. A device (1) according to claim 1, **characterized in that** the device (1) comprises at least one further pair of traction means (6) essentially attachable to opposite edge areas of the animal hide (2), the tensile stress line (9) of each pair of traction means (6) enclosing an acute angle (α) with the tension stress line (9) of an adjacent pair of traction means (6).

3. A device (1) according to any of claims 1 or 2, **characterized in that** the tension stress lines (9) of the pairs (4, 5) of traction means (6) basically intersect in a central area (10) of the animal hide (2).

4. A device (1) according to any of claims 1 to 3, **characterized in that** a distance between the first stop (11) and the second stop (12) is variable.

5. A device (1) according to any of claims 1 to 4, **characterized in that** the tensioning device (8) is connected to a cable pull (13), the cable pull (13) being designed for displacing the tensioning device (8) between the first stop (11) and the second stop (12).

6. A device (1) according to any of claims 1 to 5, **characterized in that** the tensioning device (8) comprises at least one receiving means for absorbing tension weights (14).

7. A device (1) according to claim 6, **characterized in that** the receiving means is designed as a substantially circular plate.

8. A device (1) according to claim 7, **characterized in that** the plate is arranged essentially in parallel to the bearing surface (3).

9. A device (1) according to any of claims 1 to 8, **characterized in that** the traction means (6) comprise self-locking tension clamps (7) for fastening the traction means (6) to the animal hide (2).

10. A device (1) according to any of claims 1 to 9, **characterized in that** the device (1) comprises deflection means (15) for deflecting the traction means (6).

11. A device (1) according to claim 10, **characterized in that** the deflection means (15) are arranged at an edge area of the bearing surface (3).

12. A device (1) according to claim 5, **characterized in that** the tensioning device (8) is connected to two cable pulls (13).

13. A device (1) according to any of claims 1 to 12, **characterized in that** the device (1) comprises a support frame (16), with the bearing surface (3), the traction means (6) and the tensioning device (8) being connected to the support frame (16).

14. A device (1) according to any of claims 1 to 13, **characterized in that** the bearing surface (3) is substantially circular.

15. A device (1) according to any of claims 1 to 14, **characterized in that** the bearing surface (3) comprises recesses (17), with the traction means (6), the tension clamps (7) and/or the deflection means (15) being accommodated at least partially in the recesses (17).

16. A device (1) according to any of claims 1 to 15, **characterized in that** the traction means (6) are connected to at least one hydraulic cylinder (18).

## Revendications

1. Dispositif (1) d'étirement de la peau animale (2), avec une surface de support (3) pour supporter la peau animale (2), une première paire (4) et une deuxième paire (5) de moyens de traction (6) pouvant être fixés sensiblement dans des zones périphériques opposées entre elles de la peau animale (2), dans lequel les moyens de traction (6) sont reliés avec un dispositif de tension (8) pour appliquer une contrainte de traction aux moyens de traction (6), et chaque paire (4, 5) de moyens de traction (6) forme, lors d'une application de la contrainte de traction, une ligne de contrainte de traction (9) dans la peau animale (2), la ligne de contrainte de traction (9) de la première paire (4) formant un angle aigu (α) avec la ligne de contrainte de traction (9) de la deuxième paire (5), **caractérisé en ce que** ce dispositif (1) comprend une première butée (11) et une deuxième butée (12) et que le dispositif de tension (8) est monté de façon mobile entre la première butée (11) et la deuxième butée (12), les moyens de traction (6) étant sensiblement exempts de contrainte quand le dispositif de tension (8) est appliqué contre la première butée (11) et soumis à la contrainte de traction quand le dispositif de traction (8) est appliqué contre la deuxième butée (12).

2. Dispositif (1) selon la revendication 1, **caractérisé en ce que** ce dispositif (1) comprend au moins une autre paire de moyens de traction (6) pouvant être fixés sensiblement dans des zones périphériques opposées entre elles de la peau animale (2), la ligne de contrainte de traction (9) de chaque paire de moyens de traction (6) formant un angle aigu (α) avec la ligne de contrainte de traction (9) d'une paire de moyens de traction (6) voisine.

3. Dispositif (1) selon une des revendications 1 ou 2, **caractérisé en ce que** les lignes de contrainte de traction (9) des paires (4, 5) de moyens de traction (6) se croisent sensiblement dans une zone centrale (10) de la peau animale (2).

4. Dispositif (1) selon une des revendications 1 à 3, **caractérisé en ce qu'**une distance entre la première butée (11) et la deuxième butée (12) est variable.

5. Dispositif (1) selon une des revendications 1 à 4, **caractérisé en ce que** le dispositif de tension (8) est relié à un câble (13), le câble (13) étant configuré pour déplacer le dispositif de tension (8) entre la première butée (11) et la deuxième butée (12).

6. Dispositif (1) selon une des revendications 1 à 5, **caractérisé en ce que** le dispositif de tension (8) comprend au moins un moyen de réception pour recevoir des poids de tension(14).

7. Dispositif (1) selon la revendication 6, **caractérisé en ce que** le moyen de réception est configuré comme une plaque sensiblement circulaire.

8. Dispositif (1) selon la revendication 7, **caractérisé en ce que** la plaque est disposée sensiblement parallèlement à la surface de support (3).

9. Dispositif (1) selon une des revendications 1 à 8, **caractérisé en ce que** les moyens de traction (6) comprennent des pinces de traction autobloquantes (7) pour fixer les moyens de traction (6) sur la peau animale (2).

10. Dispositif (1) selon une des revendications 1 à 9, **caractérisé en ce que** ce dispositif (1) comprend des moyens de déviation (15) pour dévier les moyens de traction (6).

11. Dispositif (1) selon la revendication 10, **caractérisé en ce que** les moyens de déviation (15) sont disposés dans une zone périphérique de la surface de support (3).

12. Dispositif (1) selon la revendication 5, **caractérisé en ce que** le dispositif de tension (8) est relié avec deux câbles (13).

13. Dispositif (1) selon une des revendications 1 à 12, **caractérisé en ce que** ce dispositif (1) comprend un cadre de support (16), la surface de support (3), les moyens de traction (6) et le dispositif de tension (8) étant reliés avec le cadre de support (16).

14. Dispositif (1) selon une des revendications 1 à 13, **caractérisé en ce que** la surface de support (3) est sensiblement de forme circulaire.

15. Dispositif (1) selon une des revendications 1 à 14, **caractérisé en ce que** la surface de support (3) comprend des cavités (17), les moyens de traction (6), les pinces de traction (7) et/ou les moyens de déviation étant au moins partiellement logés dans les cavités (17).

16. Dispositif (1) selon une des revendications 1 à 15, **caractérisé en ce que** les moyens de traction (6) sont reliés avec au moins un vérin hydraulique (18).
